# EUROPÄISCHE PATENTANMELDUNG

(11) **EP 1 688 086 A1**
(43) Veröffentlichungstag der Anmeldung: **09.08.2006**
(21) Anmeldenummer: 06000765.5
(22) Anmeldetag: 14.01.2006
(51) Int. Cl.: A61B 5/00, G08B 25/01

(54) **Vorrichtung zur Überwachung von Vitalwerten Gebrechlicher**

(30) Priorität: 08.02.2005 DE 102005006024
(71) Anmelder: Deutsche Telekom AG, 53113 Bonn (DE)
(72) Erfinder: Flügel, Gunter, 14480 Potsdam (DE); Knüpfer, Lutz, Dr., 17139 Pinnow (DE); Kröger, Holger, Dr., 17139 Malchin (DE); Stoll, Mathias, 17166 Teterow (DE)

(57) **Zusammenfassung**

Die Erfindung betrifft eine Vorrichtung zur Überwachung von Vitalwerten gebrechlicher Personen. Sie bezieht sich insbesondere auf eine Vorrichtung für den mobilen Einsatz, welche für Senioren konzipiert wurde, deren Vitalfunktionen einer Überwachung bedürfen. Die Vorrichtung umfasst Einheiten zur Erfassung und Auswertung der Vitalwerte, Einheiten zur Bestimmung des Aufenthaltsortes einer mit ihr ausgestatteten Person und einer Mobilfunksende- und Empfangseinrichtung. Bei eingeschalteter Überwachungsfunktion werden mittels der letztgenannten Einrichtung, im Falle der Feststellung mindestens eines kritischen Vitalwertes bei der vorgenannten Person, automatisch eine Notrufnummer angewählt und an diese Daten übertragen.

Erfindungsgemäß besteht die Vorrichtung zumindest aus zwei Funktionsmodulen, von denen eines zumindest die Einheiten zur Erfassung und Auswertung der Vitalwerte aufnimmt und eines als ein auch zur Sprachkommunikation geeignetes Mobiltelefon ausgebildet ist. Die genannten Funktionsmodule arbeiten als Master-Slave-Anordnung.

## Beschreibung

Die Erfindung betrifft eine Vorrichtung zur Überwachung von Vitalwerten gebrechlicher Personen. Sie bezieht sich insbesondere auf eine Vorrichtung für den mobilen Einsatz, welche für Senioren konzipiert wurde, deren Vital- beziehungsweise Lebensfunktionen aufgrund gesundheitlicher Schwierigkeiten einer Überwachung bedürfen.

Seit längerem sind Systeme bekannt, mittels derer in einer Notsituation befindliche Menschen andere Personen an entfernter Stelle auf ihre Notsituation aufmerksam machen können. Beispielhaft sei hier die aus Krankenhäusern bekannte Notsignaltaste genannt.
Insbesondere durch die gestiegene Lebenserwartung der Menschen gibt es aber vermehrt Personen, die einer ständigen stationären Behandlung nicht bedürfen und die sogar trotz gewisser gesundheitlicher Einschränkungen weitgehend mobil sind und nahezu im vollen Umfang am öffentlichen Leben teilnehmen. Häufiger unterliegen dabei insbesondere ältere Menschen gesundheitlichen Risiken, welche dazu führen können, dass sich der Gesundheitszustand unerwartet und schnell bedrohlich verschlechtert. In vielen Fällen sind derartige Schwankungen des Gesundheitszustands und des Wohlbefindens durch Erkrankungen des Herz- und Kreislaufsystems bedingt. So können etwa Herzrhythmusstörungen oder ein plötzlich ansteigender Blutdruck eine sich im öffentlichen Raum bewegende Person unerwartet vor große Probleme stellen.
Es wurden daher Lösungen entwickelt, die es gesundheitlich beeinträchtigten beziehungsweise gebrechlichen Menschen auch abseits der stationären Betreuung und des Aufenthalts in der eigenen Wohnung ermöglichen, auf eine eventuelle, sie betreffende Notsituation unter Nutzung des heute weit verbreiteten Mobilfunks aufmerksam zu machen. Derartige Lösungen finden sich beispielsweise unter www.kretschmer-berlin.com/nottel4.htm. An genannter Stelle wird zum Beispiel ein funkbasiertes Nottelefon beschrieben, welches eine spezielle Notruftaste aufweist, bei deren Betätigung eine sofortige Direktwahlverbindung zu einer Notrufnummer hergestellt wird. Nach dem Aufbau der Verbindung werden über diese Positionsdaten zum aktuellen Aufenthaltsort der das Gerät nutzenden Person übertragen. Eine vergleichbare Lösung wird auch durch die DE 198 36 118 A1 beschrieben. Die Schrift offenbart ein Gerät und System zur weltweiten Notfallrettung per Ein-Tasten-Bedienung. Hierbei handelt es sich um ein Handy mit einem, im Interesse einer einfachen Bedienbarkeit, deutlich reduzierten Funktionsumfang und einer geringen Tastenzahl. Dieses soll den Nutzer im Falle einer Notsituation, welche gesundheitlich bedingt oder durch einen Unfall, einen Überfall oder ein anderes Ereignis hervorgerufen sein kann, in die Lage versetzen, mit nur einem Tastendruck eine weltweit zentrale Notrufnummer anzurufen. Das dargestellte Handy verfügt jedoch nicht über Funktionseinheiten, welche eine sich ankündigende Verschlechterung des Gesundheitszustandes einer das Gerät nutzenden Person selbständig erkennen würden. Zudem beinhaltet die Lösung nicht die Möglichkeit einer Vorhersage (Prognose) des zukünftigen Gesundheitszustandes. Die Lösung ist somit im Grunde passiv und zeigt lediglich das Ergebnis eines Zustandes an - zum Beispiel: Der Patient hatte einen Infarkt". Eine insoweit ebenfalls vergleichbare Lösung ist außerdem aus der DE 197 15 542 A1 bekannt, welche jedoch ein Notebook als Datenerfassungsgerät einbezieht, aus dessen Speicher über das mit ihm verbundene Handy im Zuge eines Notrufs auch zuvor eingegebene Patientendaten an die gerufene Stelle übertragen werden können. Eine ebenfalls interessante Lösung wird weiterhin auf der bereits zitierten Internetseite angegeben. Diese betrifft ein Handy, welches die von einer Notsituation betroffene Person auf ihre Brust auflegen kann, wo es EKG-Daten erfasst und an eine zuvor über die Betätigung einer Notruftaste angewählte Notrufnummer überträgt. Allen zuvor genannten Lösungen ist gemeinsam, dass sie eine Bedienung, nämlich zumindest das Betätigen einer Taste, durch die in Not geratene Person erfordern, welche unter Umständen, beispielsweise aufgrund eingetretener Bewusstlosigkeit, hierzu gar nicht mehr in der Lage ist.

Darüber hinaus ist aus der DE 198 48 229 ein mobiles Gerät zur Aufzeichnung und Übertragung von digitalisierten medizinischen Daten bekannt, in welches eine GSM-Einheit integriert ist. Das System ist entweder zur Übertragung der aufgezeichneten medizinischen Daten permanent online oder kann ereignisgesteuert online geschaltet werden und entsprechende medizinische Daten unter Nutzung der integrierten GSM-Einheit per Mobilfunk an eine empfangende Stelle übertragen. Im Hinblick darauf, dass heutzutage viele Menschen, so auch ältere oder in sonstiger Weise gebrechliche Menschen, ein Mobilfunktelefon mit sich führen, ist es bei dieser Lösung als nachteilig anzusehen, dass der Nutzer des beschriebenen Geräts mit diesem eine zweite GSM-Einheit mit sich führen muss. Zudem ist durch die starre Kopplung der Einheiten zur Aufzeichnung der medizinischen Daten mit der GSM-Einheit in einem integrierten Gerät dessen Flexibilität deutlich eingeschränkt.

Der Erfindung liegt die Aufgabe zugrunde, eine Lösung zu schaffen, welche es gebrechlichen oder gesundheitlich eingeschränkten Menschen ermöglicht, ungebunden und mobil am öffentlichen Leben teilzuhaben, ihnen aber im Falle des Entstehens gesundheitlich bedingter Notsituationen schnell helfen zu können. Dabei soll die zu schaffende Lösung hinsichtlich ihres Einsatzes eine hohe Flexibilität und für den Benutzer, hinsichtlich ihrer Nothilfefunktion im Grunde kaum wahrnehmbar, einen hohen Benutzungskomfort bieten.

Die Aufgabe wird durch eine Vorrichtung mit den Merkmalen des Hauptanspruchs gelöst. Vorteilhafte Aus- beziehungsweise Weiterbildungen der Erfindung sind durch die Unteransprüche gegeben.
Die enfindungsgemäße Vorrichtung zur Überwachung der Vitalwerten gebrechlicher Personen ist als mobile Vorrichtung ausgebildet und besteht aus Einheiten zur Erfassung und Auswertung der Vitalwerte, Einheiten zur Bestimmung des Aufenthaltsortes einer mit der Vorrichtung ausgestatteten Person und einer Mobilfunksende- und Empfangseinrichtung. Bei eingeschalteter Überwachungsfunktion werden mittels der letztgenannten Einrichtung, im Falle der Feststellung mindestens eines kritischen Vitalwertes bei der vorgenannten Person, automatisch eine Notrufnummer angewählt und an diese Daten übertragen, welche die erfassten Vitalwerte der Person und ihren vermittels der Einheiten zur Ortsbestimmung bestimmten aktuellen Aufenthaltsort repräsentieren. Erfindungsgemäß besteht die Vorrichtung zumindest aus zwei Funktionsmodulen, von denen eines zumindest die Einheiten zur Erfassung und Auswertung der Vitalwerte aufnimmt und eines als ein Mobiltelefon oder Smartphone ausgebildet ist, welches im Grunde ein gewöhnliches Handy darstellt und zumindest auch zur Sprachkommunikation, vorzugsweise auch für den Versand von SMS oder gegebenenfalls E-Mail, verwendbar ist. Dabei bilden die genannten Funktionsmodule eine Master-Slave-Anordnung aus, in welcher das Mobiltelefon oder Smartphone den von dem Funktionsmodul mit den Einheiten zur Erfassung und Auswertung der Vitalwerte über eine Daten- und Signalverbindung gemasterten Slave darstellt. Im Falle der Feststellung eines kritischen Vitalwertes werden durch den Master an den Slave Daten mit den Vitalwerten der überwachten Person und zu deren augenblicklichem Aufenthaltsort sowie Steuersignale übertragen, die den Slave zum Aufbau einer Verbindung und zur Übertragung der Daten zur Notrufnummer veranlassen. Im Sinne der Erfindung handelt es sich also bei dem als Slave fungierenden Funktionsmodul um ein im Grunde gewöhnliches Handy, welches für den speziellen Einsatzzweck lediglich in seiner Funktionalität etwas erweitert wurde. Insoweit bezieht sich die Betrachtung der erfindungsgemäßen Vorrichtung als Master-Slave-Anordnung grundsätzlich nur auf den Fall der eingeschalteten Überwachungsfunktion. Dies heißt, dass das genannte Mobiltelefon von der mit der erfindungsgemäßen Vorrichtung ausgestatteten Person in völlig üblicher Art und Weise zur sprachlichen Kommunikation oder zum Versenden und Empfangen von SMS oder E-Mail verwendet werden kann. Wenn jedoch die Überwachungsfunktion eingeschaltet ist, wobei vorzugsweise die betreffende Person die Überwachungsfunktion selbst ein- und ausschalten kann, wird dieses Handy durch das andere Funktionsmodul mit den Einheiten zur Erfassung und Auswertung der Vitalwerte gemastert respektive gesteuert. Dies wirkt sich praktisch so aus, dass im Falle der Feststellung eines kritischen Vitalwertes durch die letztgenannten Einheiten das Handy beziehungsweise Mobiltelefon, unabhängig vom Willen der mit der erfindungsgemäßen Vorrichtung ausgestatteten Person, zur Übertragung von Daten zu den Vitalwerten und dem Aufenthaltsort der Person angesteuert wird. Dabei sollte eine derartige Ansteuerung auch dann geschehen, wenn die betreffende Person gerade ein Telefonat führt oder einen Telefonanruf empfängt, so dass die in diesem Moment bestehende oder im Aufbau befindliche Telefonverbindung sofort unterbrochen und eine Verbindung zu der per Konfiguration im Handy hinterlegten Notrufnummer aufgebaut wird.

Die Einheiten zur Erfassung und Auswertung der Vitalwerte sind vorzugsweise an einem Band angeordnet, welches von der mit der Vorrichtung ausgestatteten Person an einem Arm oder einem Bein getragen wird. Alternativ kommt auch die Unterbringung an einem Gürtel oder in einer von der Person zu tragenden Weste in Betracht. Entsprechend einer praxisgerechten Ausbildungsform der erfindungsgemäßen Vorrichtung sind die Einheiten zur Bestimmung des Aufenthaltsortes der die Vorrichtung nutzenden Person mit den vorgenannten Einheiten zusammen in einem von ihnen gemeinsam ausgebildeten Funktionsmodul angeordnet. Selbstverständlich ist es aber auch denkbar, die Einheiten zur Ortsbestimmung, bei denen es sich vorzugsweise um Einheiten zur Nutzung des GPS oder des Positionsbestimmungssystems Galileo" handelt, in einem gesonderten Funktionsmodul oder in dem Handy anzuordnen. Letzteres kommt insbesondere im Hinblick darauf in Betracht, dass moderne Mobilfunktelefone heute teilweise selbst mit GPS-Einheiten ausgestattet werden. Im Hinblick auf eine Nutzung mit Handys unterschiedlichster Ausstattung ist jedoch der erstgenannten Variante der Vorzug zu geben.
Grundsätzlich ist es denkbar, die Anwahl der Notrufnummer an die Über- oder Unterschreitung absolut, also unabhängig von der Person, geltender Grenzwerte für Vitalwerte zu koppeln. Dies dürfte jedoch wenig praxisgerecht sein und unter Umständen sogar Probleme aufwerfen. Daher ist die Erfindung vorzugsweise so weitergebildet, dass das Funktionsmodul mit den Einheiten zur Erfassung und Auswertung der Vitalwerte Speichermittel aufweist, in denen per Konfiguration Normalwertbereiche der Vitalwerte der jeweiligen mit der Vorrichtung ausgestatteten Person hinterlegbar sind. Dabei umfasst das entsprechende Funktionsmodul, entsprechend einer vorgesehenen Ausbildungsform der Erfindung zumindest Mittel zur Erfassung und Bewertung des Blutdrucks und/oder der Pulsfrequenz einer das Funktionsmodul tragenden Person.
Die Übertragung der Steuersignale und der Daten mit den Vitalwerten vom Master zum Slave kann drahtgebunden erfolgen, wobei letzteres zu bevorzugen ist. Entsprechend vorteilhafter Ausbildungsformen der Erfindung ist daher die Daten- und Signalverbindung zwischen dem Master und dem Slave durch eine Infrarotverbindung oder eine Kurzstreckenfunkverbindung, beispielsweise nach dem Blue Tooth Standard gegeben, für welche an den Funktionsmodulen entsprechende Schnittstellen ausgebildet sind. Beispielsweise im Hinblick auf die Möglichkeit, das Funktionsmodul mit den Einheiten zur Erfassung und Auswertung der Vitalwerte vermittels des Handys ein- oder ausschalten zu können, sind die genannten Schnittstellen vorzugsweise bidirektional ausgebildet. Darüber hinaus ist bei einer besonders vorteilhaften Ausbildungsform mit bidirektionalen Schnittstellen die Möglichkeit gegeben, das Funktionsmodul mit den Einheiten zur Erfassung der Vitalwerte über das Handy zu konfigurieren. Über ein entsprechendes Menü ist dabei beispielsweise das Messintervall einstellbar, in welchem der oder die jeweiligen Vitalwerte durch die dafür vorgesehenen Einheiten erfasst werden. Insbesondere bezieht sich aber die Möglichkeit der Konfiguration auf die Hinterlegung von Normalwertbereichen für Vitalwerte, beispielsweise im Hinblick auf einen noch zulässigen oberen und/oder unteren Blutdruckwert, der betreffenden Person, wodurch das Ansprechverhalten der Vorrichtung festgelegt wird.
Das Mobiltelefon der erfindungsgemäßen Vorrichtung ist entsprechend bevorzugter Ausführungsformen in besonderer Weise für eine komfortable Bedienung durch gebrechliche oder in ihrer Leistungsfähigkeit eingeschränkte Personen optimiert. So sind gemäß einer vorgesehenen Ausführungsform eine oder mehrere mit Sonderfunktionen belegte Tasten von den übrigen Tasten abgesetzt angeordnet und gegenüber diesen vergrößert ausgebildet, wobei deren Zahl im Hinblick auf einen guten Kompromiss zwischen Platzbedarf und einfacher Bedienbarkeit begrenzt ist und eine Ausbildung in einer gegenüber den anderen Tasten doppelten Größe in Betracht kommt. Zwar ist die Vorrichtung darauf ausgerichtet, die Alarmierung einer Notrufnummer automatisch zu veranlassen, und zwar zwangsweise insbesondere dann, wenn die zu überwachende Person beziehungsweise der Patient selbst, beispielsweise wegen Bewusstlosigkeit, nicht mehr dazu in der Lage ist. Dennoch ist es bei einer Ausführungsform, neben der bereits erwähnten Taste zum Ein- und Ausschalten der Überwachungsfunktion beziehungsweise zur Aktivierung und Deaktivierung der Einheiten für die Vitalwerterfassung und -auswertung, zusätzlich eine Taste zur manuell betätigten Veranlassung einer Übertragung von Vitalwerten an einen Arzt oder eine Pflegeeinrichtung vorgesehen. Daneben kann eine Taste vorgesehen sein, die es dem Benutzer ermöglicht, eine augenblickliche Erfassung der Vitalwerte und deren Anzeige in einem Display des Mobiltelefons zu veranlassen. Weiterhin können Tasten zur unmittelbaren Kontaktaufnahme mit der Feuerwehr, der Polizei oder beispielsweise einem speziellen Seniorenservice an dem Mobilfunkgerät exponiert angeordnet sein. Die entsprechenden Rufnummern sind ebenso wie die Rufnummer zur automatischen Übertragung der Vitalwerte in einem eventuellen Notfall vorzugsweise in dem Handy gespeichert.
Bei Ausbildungsformen für in ihrem Sehvermögen eingeschränkte Menschen sind die Tasten des Mobiltelefons oder Smartphones mit einer besonders großen Schrift bezeichnet oder taktil unterscheidbar ausgebildet. Für letzteres kommt eine Beschriftung unter Verwendung der Brailleschrift in Betracht.

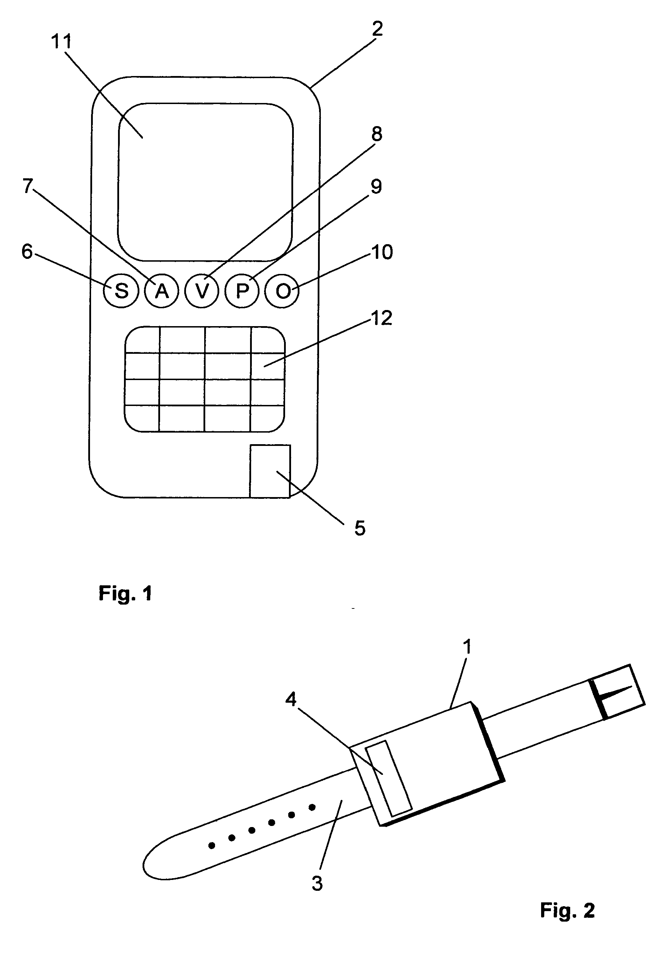

Die Erfindung soll nachfolgend in der Art eines Ausführungsbeispiels nochmals näher erläutert werden. Die zugehörigen Zeichnungen, die Fig. 1 und 2, zeigen eine mögliche Ausführungsform der erfindungsgemäßen Vorrichtung in einer stark schematisierten Darstellung.
Die Vorrichtung besteht aus zwei Funktionsmodulen 1, 2. Dabei sind die Einheiten zur Erfassung und Auswertung von Vitalwerten, wie beispielsweise des Blutdrucks oder der Pulsfrequenz, in einem an Arm oder Bein zu tragenden Band 3 untergebrachten Modul 1 angeordnet. Denkbar ist alternativ auch die Anordnung an einem Gürtel oder in einer Weste, wobei dann allerdings gegebenenfalls, beispielsweise zur Blutdruckmessung oder Pulsmessung, in nachteiliger Weise Kabel zu entsprechenden an Arm oder Bein angeordneten Sensoren vorgesehen sein müssten. Das vorgenannte Funktionsmodul 1 verfügt daneben zumindest über einen Sender 4 zur Übertragung von Daten mit den Vitalwerten der mit der Vorrichtung ausgestatteten Person und von Steuersignalen zum Handy 2, welches hierfür über einen entsprechenden Empfänger 5 verfügt. In dem dargestellten Beispiel ist in dem am Band 3 zu tragenden Modul 1 außerdem eine GPS-Einheit untergebracht. Das zweite Funktionsmodul 2 wird durch das in der Zeichnung stilisiert dargestellte Handy gebildet. Hierbei handelt es sich um ein für die gewöhnliche Sprachkommunikation verwendbares Handy 2 nach dem GSM- oder UMTS-Standard, welches darüber hinaus, wie bereits erwähnt, zumindest einen Empfänger für die von dem erstgenannten Funktionsmodul 1 ausgesandten Daten und Steuersignale und vorzugsweise, wie im dargestellten Beispiel, über weitere gesonderte Funktionstasten 6, 7, 8, 9, 10 verfügt. Mittels dieser Funktionstasten 6, 7, 8, 9, 10 kann die das Gerät benutzende Person unter anderem eine Übertragung von Daten mit Vitalwerten und/oder Positionsangaben manuell veranlassen, zum Beispiel mittels der Taste 7 eine Übertragung der Vitalwerte zu einem Arzt. Weiterhin können Tasten zur Kontaktaufnahme mit einem Seniorenservice 6 oder mit einer anderen Notrufnummer, wie der Feuerwehr oder der Polizei 9, vorgesehen sein. Im Hinblick auf die Wahrung des Rechts auf Selbstbestimmung beziehungsweise der Privatsphäre ist vorzugsweise außerdem eine Funktionstaste 8 an dem Handy 2 angeordnet, mittels welcher die automatische Überwachung der Vitalwerte abgeschaltet werden kann. Dies kann technisch dadurch geschehen, dass entweder der an dem Handy 2 vorgesehene Empfänger 5 für Daten des ersten Funktionsmoduls 1 mit den Einheiten zur Erfassung und Bewertung von Vitalwerten inaktiv geschaltet oder das letztgenannte Funktionsmodul 1 außer Betrieb gesetzt wird. Im letztgenannten Fall müssen dann allerdings die Schnittstellen für den Datenaustausch zwischen den Funktionsmodulen 1, 2 zwingend bidirektional ausgebildet sein. Auch, wenn in dem gezeigten Beispiel die Einheiten zur Erfassung und Auswertung der Vitalwerte mit denen zur Positionserfassung in einem gemeinsamen Funktionsmodul 1 angeordnet sind, ist es denkbar, dass diese mittels des Handys unabhängig von einander Schaltbar sind, im Beispiel durch die Tasten 8, 10. An dieser Stelle sei noch angemerkt, dass die Einheiten zur Positionserfassung, im Falle der Nutzung des GPS, gegebenenfalls lediglich einen GPS-Empfänger umfassen müssen und eine Auswertung der GPS-Daten möglicherweise erst in der im Notfall kontaktierten Notrufzentrale erfolgt.

### Liste der verwendeten Bezugszeichen

- 1: Funktionsmodul
- 2: Funktionsmodul, Mobiltelefon bzw. Handy oder Smartphone
- 3: Band
- 4: Schnittstelle mit Sender und ggf. Empfänger
- 5: Schnittstelle mit Empfänger und ggf. Sender
- 6, 7, 8, 9, 10: Sonderfunktionstasten
- 11: Display
- 12: Tastatur bzw. Bedienfeld

## Patentansprüche

1. Mobile Vorrichtung zur Überwachung von Vitalwerten gebrechlicher Personen, mit Einheiten zur Erfassung und Auswertung der Vitalwerte, Einheiten zur Bestimmung des Aufenthaltsortes einer mit der Vorrichtung ausgestatteten Person und einer Mobilfunksende- und Empfangseinrichtung zur Übertragung von Daten, welche die erfassten Vitalwerte der Person und ihren vermittels der Einheiten zur Ortsbestimmung bestimmten Aufenthaltsort repräsentieren, an eine Notrufnummer, wobei, bei eingeschalteter Überwachungsfunktion, eine automatische Anwahl der in der Vorrichtung hinterlegten Notrufnummer und eine Übertragung der vorgenannten Daten erfolgt, sofern durch die Einheiten zur Erfassung und Auswertung der Vitalwerte bei der genannten Person mindestens ein kritischer Vitalwert festgestellt wird, **dadurch gekennzeichnet, dass** die Vorrichtung zumindest aus zwei, voneinander abgesetzt angeordneten Funktionsmodulen (1, 2) besteht, von denen eines (1) zumindest die Einheiten zur Erfassung und Auswertung der Vitalwerte aufnimmt und eines als ein zumindest auch zur Sprachkommunikation dienendes Mobiltelefon oder Smartphone (2) ausgebildet ist und dass diese Funktionsmodule (1, 2) eine Master-Slave-Anordnung ausbilden, in welcher das Mobiltelefon oder Smartphone (2) den von dem Funktionsmodul (1) mit den Einheiten zur Erfassung und Auswertung der Vitalwerte über eine Daten-und Signalverbindung gemasterten Slave darstellt, an den der Master bei der Feststellung eines kritischen Vitalwertes Daten mit den Vitalwerten und zum augenblicklichen Aufenthaltsort der Person sowie Steuersignale überträgt, die den Slave zum Aufbau einer Verbindung und zur Übertragung der Daten zur Notrufnummer veranlassen.

2. Vorrichtung nach Anspruch 1, **dadurch gekennzeichnet, dass** das Funktionsmodul (1) mit den Einheiten zur Erfassung und Auswertung der Vitalwerte an einem von einer Person an einem Arm oder einem Bein zu tragenden Band (3) oder einer von ihr anzuziehenden Weste angeordnet ist.

3. Vorrichtung nach Anspruch 1 oder 2, **dadurch gekennzeichnet, dass** die Einheiten zur Ortsbestimmung mit den Einheiten zur Erfassung und Auswertung der Vitalwerte in einem gemeinsamen Funktionsmodul (1) angeordnet sind.

4. Vorrichtung nach Anspruch 2 oder 3, **dadurch gekennzeichnet, dass** das Funktionsmodul (1) mit den Einheiten zur Erfassung und Auswertung der Vitalwerte Speichermittel aufweist, in denen per Konfiguration Normalwertbereiche der Vitalwerte einer mit der Vorrichtung auszustattenden Person hinterlegbar sind.

5. Vorrichtung nach Anspruch 1 oder 4, **dadurch gekennzeichnet, dass** das Funktionsmodul (1) mit den Einheiten zur Erfassung und Auswertung der Vitalwerte zumindest über Mittel zur Erfassung und Bewertung des Blutdrucks und/oder der Pulsfrequenz einer es tragenden Person verfügt.

6. Vorrichtung nach einem der Ansprüche 1 bis 5, **dadurch gekennzeichnet, dass** die Daten- und Signalverbindung zwischen Master und Slave über an diesen ausgebildete Infrarotschnittstellen (4, 5) gegeben ist.

7. Vorrichtung nach einem der Ansprüche 1 bis 5, **dadurch gekennzeichnet, dass** die Daten- und Signalverbindung zwischen Master und Slave über eine Kurzfunkstreckenverbindung gegeben ist, für welche an den Funktionsmodulen (1, 2) entsprechende Schnittstellen (4, 5) ausgebildet sind.

8. Vorrichtung nach Anspruch 6 oder 7, **dadurch gekennzeichnet, dass** die Schnittstellen (4, 5) zur Daten- und Signalverbindung von Master und Slave bidirektional ausgebildet sind.

9. Vorrichtung nach Anspruch 8, **dadurch gekennzeichnet, dass** das Funktionsmodul mit den Einheiten zur Erfassung und Auswertung der Vitalwerte mittels des Mobiltelefons oder Smartphones (2) menügeführt konfigurierbar ist.

10. Vorrichtung nach Anspruch 8 oder 9, **dadurch gekennzeichnet, dass** das Funktionsmodul (1) mit den Einheiten zur Erfassung und Auswertung der Vitalwerte mittels wenigstens eines, an dem Mobiltelefon oder Smartphone (2) angeordneten Bedienelementes (8) aktivierbar oder deaktivierbar ist.

11. Vorrichtung nach einem der Ansprüche 8 bis 10, **dadurch gekennzeichnet, dass** an dem Mobiltelefon oder Smartphone (2) ein Bedienelement (7) angeordnet ist, durch dessen Betätigung eine Übertragung von Vitalwerten zu der Notrufnummer oder einen Arzt durch eine mit der Vorrichtung ausgestattete Person manuell veranlasst werden kann.

12. Vorrichtung nach einem der Ansprüche 8 bis 11, **dadurch gekennzeichnet, dass** an dem Mobiltelefon oder Smartphone (2) ein Bedienelement angeordnet ist, durch dessen Betätigung eine sofortige Erfassung der Vitalwerte veranlasst wird und diese, unabhängig von einer etwaigen Übertragung an eine Notrufnummer, für den Benutzer der Vorrichtung auf einem Display (11) des Mobiltelefons oder Smartphones (2) angezeigt werden.

13. Vorrichtung nach einem der Ansprüche 1 bis 11, **dadurch gekennzeichnet, dass** das Mobiltelefon oder Smartphone (2) mit zusätzlichen Sonderfunktionstasten (6, 7, 8, 9, 10) ausgestattet ist, welche von den übrigen Tasten der Tastatur (12) abgesetzt angeordnet und gegenüber diesen vergrößert, vorzugsweise in doppelter Größe ausgebildet sind.

14. Vorrichtung nach Anspruch 12, **dadurch gekennzeichnet, dass** die Sonderfunktionstasten (6, 7, 8, 9, 10) mindestens eine Taste (9) zur Direktwahl zu einer Notrufnummer, wie Feuerwehr und Polizei umfasst.

15. Vorrichtung nach einem der Ansprüche 1 bis 13, **dadurch gekennzeichnet, dass** die Tasten des Mobiltelefons oder Smartphones (2) taktil unterscheidbar ausgebildet sind.

16. Vorrichtung nach Anspruch 14, **dadurch gekennzeichnet, dass** die Tasten des Mobiltelefons oder Smartphones (2) mit Brailleschrift beschriftet sind.
